(19) Europäisches Patentamt
European Patent Office
Office européen des brevets

(11) EP 0 811 386 B1

(12) **EUROPEAN PATENT SPECIFICATION**

(45) Date of publication and mention of the grant of the patent:
**29.09.2004 Bulletin 2004/40**

(51) Int Cl.[7]: **A61K 47/48**, A61K 31/495

(21) Application number: **97302821.0**

(22) Date of filing: **24.04.1997**

(54) **Method of selecting a salt for making an inclusion complex**

Verfahren zur Selektion eines Salzes zur Herstellung eines Inklusionskomplexes

Méthode de sélection d'un sel pour fabrication d'un complexe d'inclusion

(84) Designated Contracting States:
**AT BE CH DE DK ES FI FR GB GR IE IT LI LU NL PT SE**

(30) Priority: **07.05.1996 US 16866**

(43) Date of publication of application:
**10.12.1997 Bulletin 1997/50**

(73) Proprietor: **PFIZER INC.**
**New York, N.Y. 10017 (US)**

(72) Inventor: **Kim, Yesook**
**Branford, Connecticut 06405 (US)**

(74) Representative: **Wood, David John et al**
**Pfizer Limited**
**European Patents Department,**
**Ramsgate Road,**
**Sandwich, Kent CT13 9NJ (GB)**

(56) References cited:
**WO-A-97/41896**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 811 386 B1

**Description**

FIELD OF THE INVENTION

This invention relates to a method of selecting a salt of a medicinal compound for use in making a composition of matter comprising said salt and a cyclodextrin. In particular, it relates to a method of locating salts which are highly soluble in aqueous cyclodextrin solution.

BACKGROUND OF THE INVENTION

Formulation of pharmaceutical dosage forms is frequently hampered by poor aqueous solubility and/or stability of the drug of interest, which in turn can severely limit its therapeutic application. Conversely, increasing drug solubility and stability through appropriate formulation can accordingly lead to increased therapeutic efficiency of the drug. Various methods have been used to increase the solubility and stability of drugs such as the use of organic solvents, emulsions, liposomes and micelles, adjustments to pH and the dielectric constant of formulations solvent systems, chemical modifications, and complexation of the drugs with appropriate complexing agents such as cyclodextrins.

Cyclodextrins, sometimes referred to as Schardinger's dextrins, were first isolated by Villiers in 1891 as a digest of Bacillus amylobacter on potato starch. The foundations of cyclodextrin chemistry were laid down by Schardinger in the period 1903-1911. Until 1970, however, only small amounts of cyclodextrins could be produced in the laboratory and the high production cost prevented the usage of cyclodextrins in industry. In recent years, dramatic improvements in cyclodextrin production and purification have been achieved and cyclodextrins have become much cheaper, thereby making the industrial application of cyclodextrins possible.

Cyclodextrins are cyclic oligosaccharides with hydroxyl groups on the outer surface and a void cavity in the center. Their outer surface is hydrophilic, and therefore they are usually soluble in water, but the cavity has a lipophilic character. The most common cyclodextrins are α-cyclodextrin, β-cyclodextrin and γ-cyclodextrin, consisting of 6, 7 and 8 α-1,4-linked glucose units, respectively. The number of these units determines the size of the cavity.

Cyclodextrins are capable of forming inclusion complexes with a wide variety of hydrophobic molecules by taking up a whole molecule, or some part of it, into the void cavity. The stability of the complex formed depends on how well the guest molecule fits into the cyclodextrin cavity. Common cyclodextrin derivatives are formed by alkylation (e.g. methyl-and-ethyl-β-cyclodextrin) or hydroxyalkylation of the hydroxyethyl-derivatives of α-, β-, and γ-cyclodextrin) or by substituting the primary hydroxyl groups with saccharides (e.g. glucosy-and maltosyl-β-cyclodextrin). Hydroxypropyl-β-cyclodextrin and its preparation by propylene oxide addition to β-cyclodextrin, and hydroxyethyl-β-cyclodextrin and its preparation by ethylene oxide addition to β-cyclodextrin, were described in a patent of Gramera et al. (U.S. Pat. No. 3,459,731, issued Aug. 1969) over 20 years ago.

Although cyclodextrins have been used to increase the solubility, dissolution rate and/or stability of a great many compounds, it is also known there are many drugs for which cyclodextrin complexation either is not possible or yields no advantages. See J. Szejtli, Cyclodextrins in Drug Formulations: Part II, Pharmaceutical Technology, 24-38, August, 1991.

Many medicinal compounds, when salt formation is feasible, are administered in the form of one or another of their pharmaceutically acceptable salts. Not all such salts are freely soluble in aqueous media, however, and accordingly complexation of the salt of interest with a cyclodextrin is often explored as a means to increase the salt's aqueous solubility. It is conventionally believed that a salt of a drug dissolves in a cyclodextrin-containing aqueous media by simply dissociating to form a charged drug molecule and a counter-ion, and that the dissociated (i.e., charged) drug molecule is the guest moiety which forms the inclusion complex with the cyclodextrin. A consequence of this is the belief that there are no differences in equilibrium solubility among the salts of a given drug in a specific cyclodextrin. Thus, if a solubility-phase diagram is generated for a particular drug in a particular aqueous cyclodextrin (i.e., a plot of the maximum equilibrium solubility of a drug salt in the aqueous cyclodextrin as a function of cyclodextrin concentration), different salts of the drug should plot out as lines having the same slope.

Summary Of The Invention

This invention provides a method of selecting, choosing, or locating one or more salts of a compound, said salts having a solubility in a cyclodextrin equal to or greater than a desired target solubility, comprising obtaining a series of salts of said compound, measuring the equilibrium solubility of each salt in said series in an aqueous solution of said cyclodextrin, and comparing each measured solubility with said target solubility. Those salt(s) having an equilibrium solubility greater than the desired target solubility are thus chosen to be the desired salt(s).

Those familiar with using cyclodextrins will appreciate that the invention has applicability to any medicinal compound which will form salts, which will form a complex with a cyclodextrin and which has poor aqueous solubility.

In a similar aspect, this invention provides a method of determining a useful salt, from within a series of salts of a particular medicinal compound, for use in making a composition of matter comprising said salt and a cyclodextrin, said method comprising:

a. obtaining said series of salts;
b. determining the equilibrium solubility, in aqueous cyclodextrin solution, of each of said salts in said series; and
c. selecting, as said useful salt, a salt in said series having a solubility in said cyclodextrin solution equal to or greater than a desired target solubility.

The invention further provides a composition of matter comprising a pharmaceutically acceptable salt of a medicinal compound and a cyclodextrin, said salt having been located or chosen by the methods above. In a preferred embodiment, the composition is an inclusion complex of a salt complexed in a cyclodextrin.

The phrase "composition of matter" as used herein encompasses, *inter alia,* compositions of a medicinal compound and a cyclodextrin which are dry physical mixtures, which are dry inclusion complexes, and which are aqueous solutions of dissolved inclusion complexes. For example, the composition can comprise a dry mixture of a medicinal compound physically mixed with a dry cyclodextrin. The composition, in a preferred embodiment, can also comprise an aqueous solution which has been lyophilized or otherwise dried, for example in a vacuum oven or other suitable device, such that the composition comprises a (pre-formed) inclusion complex of cyclodextrin-complexed compound which can later be re-constituted. The composition can also comprise the solution itself, i.e., a medicinal compound plus cyclodextrin plus water. Inclusion complexes are within the scope of the term "composition of matter' whether they are pre-formed, formed *in situ,* or formed *in vivo.*

In a more particular embodiment, this invention provides a method of determining a useful salt, from within a series of salts of a particular medicinal compound, for use in making a composition of matter comprising an inclusion complex of said salt in a cyclodextrin, said method comprising:

a. determining or choosing a quantity of said medicinal compound required for therapeutic efficacy;
b. determining or choosing a maximum total dose in which to administer said quantity of medicinal compound;
c. calculating the minimum required solubility of a salt of said compound necessary to formulate said maximum total dose;
d. obtaining said series of salts;
e. determining the equilibrium solubility of each of said salts in said cyclodextrin; and
f. selecting, as said useful salt, a salt from said series having a solubility in said cyclodextrin sufficient to permit making a total dose equal to or less than said maximum total dose.

Reference above to a "series of salts" of a compound means, of course, that the compound must be capable of salt formation. Further, the terminology a "series of salts of a particular medicinal compound" means any two or more different salts of a particular medicinal compound. The series can be assembled as a group and tested side-by-side" to determine whether any of the salts are useful for making a useful salt/cyclodextrin composition, or each member of the group can be tested separately, for example at different times and in different locations. The series of salts can be "obtained" in any manner, for example by making them or ordering them pre-made from a commercial suppplier. The term "salt" generally means a pharmaceutically acceptable salt. The salt can be anhydrous or in the form of one or more solvates, such as hydrates, including mixtures thereof. The salts may occur in different polymorphic forms.

A "desired target solubility" as used herein can be a mimimum solubility, usually pre-determined or pre-chosen, required for the compound being tested. The required minimum solubility will generally be chosen on the basis of therapeutic need. For example, assume that it is desired to administer 20 mg of a compound ("Compound X") parenterally, by injection, and that it is desired to administer an injection volume of not more than 2 ml to minimize pain on injection. Thus a salt of Compound X, in order to be "useful", would need to have a solubility, in the chosen aqueous cyclodextrin, equivalent to or greater than 10 mg/ml of Compound X in its active form.

Within a given series of salts, the most soluble salt may not be the most useful candidate for a given application. Factors such as chemical stability, hygroscopicity, and the potential for precipitation may also be considered and weigh in favor of choosing a candidate having a solubility greater than the target solubility, but less than the maximum determined within the series.

On the other hand, at times it may indeed be desired simply to find the salt with the highest solubility of all salts within a series of salts of a particular compound. In this case the "desired target solubility" is simply the highest solubility encoutered in the series of salts by comparison of equilibrium solubilities among the various salt candidates. For example, if it is desired to make a dry oral dosage form such as a capsule or tablet using an inclusion complex of a salt of Compound X, then it may be desired simply to find the most soluble salt available in order to minimize the amount of inclusion complex in the dosage form, and thereby minimze the size of the dosage form itself.

"Maximum total dose" means the intended maximum size of a dose, including excipients and liquids (e.g., for an injectable) which are to be included in a dosage form, considering the patient or patient population for which the dosage form is meant. Typically, a maximum total dose for an injectable is considered to be about 2 ml for adults. A maximum total dose for a tablet or capsule is typically a couple of grams to ensure the dosage form is swallowable. Sizes, weights and volumes are "intended", meaning that they can change or shift depending on the particular patient population.

This invention is based, *inter alia,* on the discovery that for a particular cyclodextrin, the solubility of a particular compound in an aqueous solution of a cyclodextrin is not independent of the salt employed. That is, different salts of the same compound can often exhibit widely differing solubilities in the same cyclodextrin. The phenomenon of differential solubility exhibited by different salts of a compound in the same cyclodextrin has not heretofore been known in the art. It has also been determined that the rank order of solubility, that is the increasing or decreasing order of solubility of a series of salts in an aqueous cyclodextrin solution does not necessarily correlate with the order of salt solubility in water.

The discovery of such differential solubility of different salts in a particular cyclodextrin is surprising and unexpected based on conventional wisdom which teaches that the total solubility of an ionizable compound in a cyclodextrin-containing aqueous solution is the sum total of the solubility of all the species of the compound that exists in various forms in the solution. In a cyclodextrin-containing solution this may be represented by the following expression:

Total Drug Solubility = Fraction of free drug in unionized form + Fraction of complexed drug in unionized form + Fraction of charged drug in free form + Fraction of charged drug in complexed form

Further, it is conventionally believed that a salt form of an ionizable compound dissolves in an aqueous solution by dissociating completely according to its solubility product, as described by expression

$$(DH\,X) \overset{ksp}{\rightleftharpoons} DH^{+} + X^{-}$$

where

- $DH^{+} \overset{Ka}{\rightleftharpoons} D + H^{+}$,
- DHX is the acid addition salt of a basic compound,
- $DH^{+}$ is the charged form in solution,
- $X^{-}$ is the counter ion,
- D is the unionized form in solution,
- $H^{+}$ is the proton concentration dictated by the pH of the solution, and
- Ka is the dissociation constant.

Hence, for a particular compound various salt forms are expected to have different aqueous solubilities dictated by the Ksp. The above expressions further indicate that at a constant ionization state (i.e. constant pH), the difference in solubility among various salt forms for a particular compound should be same both in the presence and absence of a particular cyclodextrin. Hence, if a phase solubility diagram is generated for a particular compound in an aqueous solution containing a particular cyclodextrin as a function of cyclodextrin concentration, different salts of the compound should plot out as lines having different intercepts, but having the same slope.

Thus, based on conventional belief there is no reason to expect that different salts of a particular compound would be differentially solubilized in the same cyclodextrin since it is believed that the counter ion does not play a role in the complexation process.

Further, the phenomenon of differential solubility is important because it makes possible the capability for increasing the loading of a particular compound in a cyclodextrin by testing a series of different salts of that compound and selecting a salt which affords a desired high solubility, thereby permitting the use of a lower amount of cyclodextrin relative to a less cyclodextrin-soluble salt. The phenomenon is particularly important in the case of parenteral administration ( i.e., by injection) because, assuming a constant concentration of inclusion complex in water, injection volume can be reduced by choosing an appropriate highly cyclodextrin-soluble salt. As noted above, by locating highly cyclodextrin-soluble salts, the invention also provides an opportunity to reduce the size of dry dosage forms (such as tablets

and capsules) by using correspondingly lower amounts of inclusion complex relative to the amounts of inclusion complex for less cyclodextrin-soluble salts.

Brief Description Of The Drawings

Figure 1 is a solubility phase diagram which is a plot of the maximum equilibrium solubility of a series of salts of the compound ziprasidone as a function of SBECD concentration in water.
The ordinate (Y-axis) is Drug Solubility (units are millimolar) and the abscissa (X-axis) is SBECD concentration (also millimolar units).
The symbology employed is explained in the following chart:

| Salt | |
|---|---|
| + | Mesylate |
| X | Tartrate |
| Δ | Esylate |
| • | Napsylate |
| ○ | HCl |

Detailed Discussion

The amount of medicinal compound to be administered to a patient is an effective amount. The amount, mode of administration such as oral, parenteral, and so forth, and dosing regimen (e.g., whether the dose is to be divided and frequency of administration) will of course vary with the compound being administered, the patient population, and so forth. The amount of cyclodextrin used in a particular formulation will be a bioavailability-increasing amount. Small amounts of cyclodextrin even when present in a dosage form which is a mixture, can enhance bioavailability by forming an inclusion complex *in vivo,* thus increasing the bioavailability of the drug relative to uncomplexed drug. Generally the amount of cyclodextrin in a formulation is usually such that the molar ratio of cyclodextrin to drug is between 0.1:1 and 100:1, preferably between 0.25:1 and 10:1, more preferably between 0.5:1 and 5:1. If the formulation is an aqueous solution, it can contain cyclodextrin in a wide range of concentrations, e.g., from 5 wgt % (w/v) to over 100 wgt % (w/v). At high concentrations of cyclodextrins, formulations become somewhat viscous and are amenable to oral administration as elixirs or syrups.
The invention is applicable to cyclodextrins in general, including those which are presently known. Useful cyclodextrins include α, β, and γ cyclodextrins, methylated cyclodextrins, hydroxypropyl-β-cyclodextrin (HPBCD), hydroxyethylated-β-cyclodextrin (HEBCD), branched cyclodextrins in which one or two glucoses or maltoses are enzymatically attached to the cyclodextrin ring, ethyl- and ethyl-carboxymethyl cyclodextrins, dihydroxypropyl cyclodextrins, and sulfoalkyl ether cyclodextrins. The degree of substitution is not considered to be critical, and the cyclodextrins just mentioned can have essentially any degree of substitution (per entire cyclodextrin molecule) known in the art. Mixtures of cyclodextrins, as well as single species, are feasible for making dosage forms according to the invention.
HPBCD is well known in the art, see for example Publication R 81 216 entitled "Encapsin HPB" from Janssen Biotech N.V.. SBECD is also known and has been disclosed in U.S. Patents 5,376,645 and 5,134,127, both to Stella et al. and both herein incorporated by reference in their entirety.
The pharmaceutically acceptable acid or base addition salts of a compound capable of salt formation can be prepared as known in the art by conventional methodology by treating a solution or suspension of the compound with about one chemical equivalent of a pharmaceutically acceptable acid or base, as appropriate, depending of course on whether the compound forms acid addition salts or base addition salts. The salt can be isolated by conventional methods, such as by filtration when the salt spontaneously precipitates (e.g., as a crystalline material), or it can be otherwise isolated by concentration and/or addition of a non-solvent. For example, the salts employed in the Examples below were made by first weighing an amount of ziprasidone free base and adding it to a solvent, typically an organic solvent, water, or a mixture of two or more solvents. The solvent(s) used can depend on whether it is desired to isolate the salt from a slurry or from a solution. If it is desired to isolate the salt from a solution, the solvent can be heated, with stirring, to facilitate dissolution. About one molar equivalent of an acid or base, as appropriate, or a slight excess, corresponding to the desired counterion is added with stirring. After a period of time which can be determined by simple experimentation, typically hours, the solids can be harvested by filtration and washed.
An inclusion complex of a pharmaceutically acceptable salt of a compound can be formed conventionally by known methodology. That is, an inclusion complex of a desired pharmaceutically acceptable salt can be formed *in situ*

by adding the salt directly to a pre-made solution of cyclodextrin in water (or other suitable pharmaceutically acceptable aqueous medium) in an amount sufficient to make a product solution of the desired strength. Alternatively, the drug and cyclodextrin can be added to the water separately or together as a mixture. The product solution can be used immediately or stored (at room temperature or at reduced temperature) depending on the shelf life of the inclusion complex. A pharmaceutically acceptable preservative or other excipients may be added to render the dosage form stable to chemical, physical, or microbial degradation. If SBECD is employed as the cyclodextrin, since SBECD is generally used in the form of its sodium salt, the product solution can be used as is (with rewarming to room temperature if the solution was stored) for administration to patients, no adjustment to isotonicity being required. If isotonicity needs to be adjusted, it can be adjusted as known in the art by adding an appropriate amount of an isotonicity adjusting agent.

Alternatively, the inclusion complex of a salt in aqueous cyclodextrin cyclodextrin can first be isolated, usually by lyophilization. The isolated inclusion complex can be stored at room temperature during its shelf life (usually at least two years) and made up into a product solution as needed. When a product solution is required, it can be made by dissolving the isolated inclusion complex in water or other aqueous medium in an amount sufficient to generate a solution of the required strength for oral, parenteral or other route of administration to patients. If necessary to adjust isotonicity, it can be accomplished conventionally as known in the art by adding an isotonicity adjusting agent.

Alternatively, a solid physical mixture comprising a salt of a drug and a cyclodextrin can be made in the form of a tablet or capsule which dissolves in gastrointestinal fluids after oral ingestion. Such mixtures may also be incorporated into buccal, sublingual, nasal, topical, or transdermal dosage forms. Such compositions may also be incorporated as solutions or suspensions in soft-gelatin capsules.

The phenomemon of different solubilities for different salts in a given cyclodextrin is general. The invention is not limited to any particular compound, class of compounds, or to any particular cyclodextrin. Rather the invention is applicable to salts generally. Moreover, the invention is not limited to any particular dosage form or route of administration. Rather, the invention is useful whenever increased solubility of a salt of a compound is desired.

For purposes of illustration, the following discussion is directed to a particular compound, ziprasidone, which has the structure

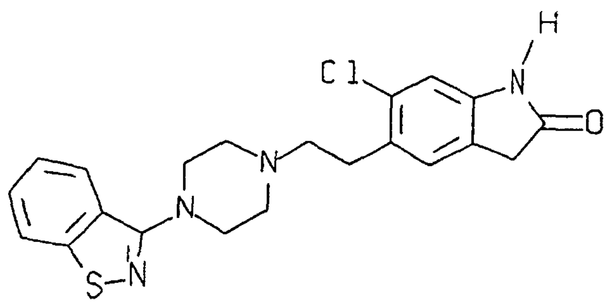

It is disclosed in the U.S. patent 4,831,031, has utility as a neuroleptic, and is thus useful as an antipsychotic. Those skilled in the art will, of course, recognize that the teachings with respect to salts of ziprasidone are applicable to other salts generally as well.

Solubility testing of various ziprasidone salts in cyclodextrin (SBECD and HPBCD) was conducted by comparing the maximum equilibrium solubility of each salt in an equal amount of cyclodextrin. Many different experimental protocols can be envisioned and implemented. The following protocol employing 40% aqueous cyclodextrin as a standard solution for comparison of equilibrium salt solubilities, but that concentration is not to be considered as limited. Other concentrations can be employed as well for purposes of serving as a comparison standard. The HPBCD employed was purchased commercially from Wacker Chemie. The SBECD employed had a degree of substitution with sulfobutyl groups of 6.5, average, per molecule of β-cyclodextrin, made by a process along the lines of that described in Example 3 of U.S. Patent 5,376,645.

A 40% (w/v) solution of cyclodextrin (SBECD or HPBCD) in water was prepared by adding 200 g of cyclodextrin to a 500 mL beaker containing approximately 250 mL of deionized water and a magnetic stir bar. The contents were stirred until dissolution of the cyclodextrin in the water was complete, usually a time of about one hour being sufficient. The solution was then transferred to a 500 mL volumetric flask and deionized water was added to the mark. 5 mL of the volumetric solution was pipetted into a 10 mL glass vial with a screw cap. An excess of the solid ziprasidone salt test candidate and a magnetic stir bar were added to the vial. The vial contents were stirred for four days at ambient temperature to allow a sufficient time for equilibrium to be reached. Upon removal from the magnetic stirrer, the sample had undissolved solid present, indicating a saturated solution under the conditions employed. The contents were filtered into a clean screw cap vial through a Millex-GS 0.2 μm filter and the drug concentration determined by an HPLC method.

As an example of an HPLC assay, the amount of dissolved compound can be determined by using a C18 Puresil (Registered Trademark of Waters Associates) column with an isocratic mobile phase consisting of 60% 0.05 M potassium dihydrogen phosphate buffer and 40% methanol, at a flow rate of 2 mL/min at 40 °C. Detection can be by UV absorption at a wavelength of 229 nm. Quantification can be effected facilely by comparison of HPLC peak height (or area) with the peak height (or area) taken from a standard plot of concentration vs. peak height (or area) for standards of known concentration. As is conventional, the ziprasidone standard concentrations are selected to fall within a linear range of concentration vs absorbance for the UV detector employed. The saturated equilibrium solution obtained after filtering the vial test solution may need to be diluted in serial fashion to reach the linear range of the standard plot, and dilution can be effected by adding isocratic mobile phase.

The above procedure was also employed to determine the solubility of ziprasidone in other concentrations of cyclodextrin. By doing this and using the data to make solubility phase diagrams for different ziprasidone salts, it was determined that the solubility phase diagrams were linear for each salt, but that the slopes were different, thereby demonstrating that different ziprasidone salts can have different equilibrium solubilities in the same cyclodextrin. The solubility phase diagram generated by doing this for different ziprasidone salts is shown in Figure 1.

Using the above HPLC procedure (including the column and isocratic mobile phase) a number of ziprasidone salts were tested to determine the equilibrium solubility of each in 40% HPBCD and in 40% SBECD. Results are reported in Table 1.

Table I:

| Solubility of ziprasidone salts in water and 40% cyclodextrin solutions. | | | |
|---|---|---|---|
| Salt form | Solubility in water | Solubility in 40% HPBCD | Solubility in 40% SBECD |
| free base | 0.3 μgA/ml | 0.26 mgA/ml | 0.35 mgA/ml |
| tosylate | 5 μgA/ml | NT | 14mgA/ml |
| napsylate | 34 μgA/ml | NT | 14 mgA/ml |
| besylate | 80 μgA/ml | NT | 12 mgA/ml |
| hydrochloride | 80 μgA/ml | 2.4 mgA/ml | 4 mgA/ml |
| aspartate | 170 μgA/ml | 1.3 mgA/ml | 9.3 mgA/ml |
| tartrate | 180 μgA/ml | 12.4 mgA/ml | 26 mgA/ml |
| esylate | 360 μgA/ml | 13.7 mgA/ml | 15 mgA/ml |
| mesylate | 1000 μgA/ml | 17.3 mgA/ml | 44 mgA/ml |
| Note: mgA indicates the weight (in mg) of ziprasidone calculated as the free base, Molecular weight=412.9; NT = Not tested<br>Molecular weight of β-cyclodextrin sufobutyl ether (SBECD): 2163; 40% (w/v) = 400 g/L = 0.18 M;<br>Molecular weight of hydroxy propyl β-cyclodextrin (HPBCD): 1309; 40% (w/v) = 400 g/L = 0.31 M | | | |

As previously mentioned, the order of solubility of a series of salts in water does not necessarily parallel the order of solubility in aqueous cyclodextrin solution. Table 1 illustrates this point. For example, the esylate salt of ziprasidone is twice as soluble in water as the tatrate. The solubility for these same two salts is roughly the same in aqueous HPBCD, and reversed in aqueous SBECD.

Table 1 indicates that for the particular ziprasidone salt candidates and cyclodextrin solutions tested, the highest solubility of ziprasidone can be achieved by dissolving ziprasidone mesylate in 40% SBECD. To deliver a therapeutic dose of ziprasidone of 80 mg/day of ziprasidone to a patient, the volume of 40% solution needed can be calculated as follows:

$$80 \text{ mgA/day} \times 1 \text{ ml}/44 \text{ mgA} = 1.8 \text{ ml/day}$$

Thus with the instant invention, as exemplified above specifically for salts of ziprasidone, therapeutically useful salt inclusion complexes, that is inclusion complexes which deliver a desired therapeutic dose of a compound, can be located.

As seen from Figure 1, ziprasidone salt solubility is linear as a function of cyclodextrin concentration in water. This illustrates that the maximum amount of a particular salt which can be dissolved in an aqueous cyclodextrin can

be measured as known in the art directly from such a solubility phase diagram (i.e., employing the appropriate line as a calibration plot), or calculated if the slope (and y-intercept, if it is non-zero) of the appropriate line has been computed.

As previously mentioned, the inclusion complex can be formulated for oral or for parenteral administration, usually intramuscular administration, to a patient. Subcutaneous and intravenous administration is also feasible. The inclusion complexes can also be administered orally in conventional forms, for example, as tablets, capsules, powders for oral suspensions, and unit dose packets containing a single dose (referred to in the art as a "sachet"). They can also be administered as buccal or sublingual tablets, as nasal sprays, in topical creams, in transdermal patches, and as suppositories.

The following examples further disclose and illustrate the invention:

Examples 1 and 2 illustrate the invention with ziprasidone.

**EXAMPLE 1**

A 300 mg/ml SBECD solution is prepared by dissolving SBECD in a pharmaceutically acceptable aqueous medium such as water. Ziprasidone mesylate is dissolved in the SBECD solution to make a concentration of 27.3 mg/ml (20 mgA/ml). The solution is sterile filtered through a 0.2 µm filter. Glass vials are filled with the filtered solution to make a product solution which can be administered orally or by an intramuscular, intravenous, or subcutaneous route.

**EXAMPLE 2**

A product solution is made as described in Example 1. Glass vials containing product solution are loaded into a freeze dryer and the product solution is freeze dried. The vials and their lyophilized contents are stored at room temperature until needed, at which time they are reconstituted with water or a pharmaceutically acceptable aqueous buffer for administration orally or by an intramuscular, intravenous, or subcutaneous route.

The following examples illustrate how to calculate dosage levels for particular inclusion complexes to deliver a particular dose, and also how to minimize injection volume.

**EXAMPLE 3**

Compound A, a poorly soluble (in water) drug, is a carboxylic acid having a molecular weight of 350. It is administered in a preferred dose of 75 mgA/day for adults ("mgA" meaning milligrams of active compound, the free acid) and 25 mgA/day for children. The following series of base addition salts has the solubilities indicated for each in 40% (w/v) aqueous cyclodextrin:

| free acid | 2 mgA/ml |
|---|---|
| Salt A | 13 mgA/ml |
| Salt B | 38 mgA/ml |
| Salt C | 52 mgA/ml |
| Salt D | 37 mg A/ml |
| Salt E | 5 mgA/ml |

A target volume, for administration as an injectable, of not more than 2ml for adults and not more than 0.5 ml for children is established. It is determined that Salt B (2.0 ml injection to deliver 75 mgA) and Salt C (1.4 ml injection volume to deliver 75 mgA) are suitable for adults. It is determined that only salt C is suitable for children (0.48 ml to deliver 25 mgA) since all other salts require more than 0.5 ml to deliver 25 mg.

**EXAMPLE 4** - Ziprasidone mesylate

1 g of ziprasidone free base was added to 20 mL of isopropyl alcohol, followed by 140mg of methanesulfonic acid. After a few minutes the slurry which formed thickened and lightened somewhat in color as it precipitated. The salt was harvested by filtration through a 5 µm polytetrafluoroethylene membrane.

**EXAMPLE 5** - Ziprasidone esylate

1 g of ziprasidone free base was added to 45 mL of THF and 1 mL of water, and the mixture was heated to 60 °C while stirring. The mixture was maintained at 60 °C for two hours, at which time all of the free base had dissolved.

156 mg of ethanesulfonic acid was added and stirring was maintained at 60 °C for two more hours. The mixture turned from light orange to hazy during this time, at which point heating was stopped and the salt started to precipitate. The mixture was allowed to cool to room temperature overnight while stirring continued. The salt was then harvested by filtration as in Example 5.

**EXAMPLE 6** - Ziprasidone tartrate

1 g of ziprasidone free base was added to 60 mL of water and the resulting slurry was heated to 50 °C for 3 hours with stirring. 900 mg of L-tartaric acid was added. Heating at 50 °C and stiring were continued for 6 more hours, and then the mixture was stirred at 40 °C overnight. The solution was then allowed to cool and the salt harvested as in Example 5.

## Claims

**1.** A method of locating one or more salts of a compound, said salts having a solubility in a cyclodextrin equal to or greater than a desired target solubility, comprising obtaining a series of salts of said compound, determining the equilibrium solubility of each salt in said series in an aqueous solution of said cyclodextrin, and comparing each measured solubility with said target solubility.

**2.** A method of determining a useful salt, from within a series of salts of a particular medicinal compound, for use in making a composition of matter comprising said salt and a cyclodextrin, said method comprising:

a. obtaining said series of salts;
b. determining the equilibrium solubility, in aqueous cyclodextrin solution, of each of said salts in said series; and
c. selecting, as said useful salt, a salt in said series having a solubility in said cyclodextrin solution equal to or greater than a desired target solubility.

**3.** A method of determining a useful salt, from within a series of salts of a particular medicinal compound, for use in making a composition of matter comprising an inclusion complex of said salt in a cyclodextrin, said method comprising:

a. determining a quantity of said medicinal compound required for therapeutic efficacy;
b. choosing a maximum total dose in which to administer said quantity of medicinal compound;
c. calculating the minimum required solubility of a salt of said compound necessary to formulate said maximum total dose;
d. obtaining said series of salts;
e. determining the equilibrium solubility of each of said salts in said cyclodextrin; and
f. selecting, as said useful salt, a salt from said series having an equilibrium solubility in said cyclodextrin sufficient to permit making a total dose equal to or less than said maximum total dose.

## Patentansprüche

**1.** Ein Verfahren zur Auffindung eines oder mehrerer Salze einer Verbindung, wobei besagte Salze eine Löslichkeit in einem Cyclodextrin aufweisen, die gleich oder größer als die gewünschte Löslichkeit ist, die als Ziel vorgegeben ist, umfassend Erhalt einer Serie von Salzen besagter Verbindung, die Bestimmung des Löslichkeitsgleichgewichtes eines jeden Salzes besagter Reihe in einer wässrigen Lösung besagten Cyclodextrins und der Vergleich jeder gemessenen Löslichkeit mit besagter Löslichkeit, die als Ziel vorgegeben ist.

**2.** Ein Verfahren zur Bestimmung eines nützlichen Salzes aus einer Serie von Salzen eines bestimmten Arzneistoffes zur Verwendung bei der Herstellung einer Komposition von Stoffen, die besagtes Salz und ein Cyclodextrin umfassen, wobei besagtes Verfahren umfasst:

a. Erhalt besagter Serien von Salzen;
b. Bestimmung der Gleichgewichtslöslichkeit eines jeden der besagten Salze in besagten Serien in wässriger Cyclodextrinlösung und

c. Selektion besagten nützlichen Salzes, eines Salzes besagter Reihe, das in besagter Cyclodextrinlösung eine Löslichkeit aufweist, die gleich oder größer ist als die der gewünschten Löslichkeit, die als Ziel vorgegeben ist.

**3.** Ein Verfahren zur Bestimmung eines nützlichen Salzes aus einer Serie von Salzen eines bestimmten Arzneistoffes zur Verwendung bei der Herstellung einer Komposition von Stoffen, das einen Einlagerungskomplex des besagten Salzes in Cyclodextrin umfasst, wobei besagtes Verfahren umfasst:

a. Bestimmung der zur therapeutischen Wirksamkeit notwendigen Menge des besagten Arzneistoffes;
b. Auswahl der maximalen Gesamtdosis, die von besagter Menge an Arzneistoff verabreicht wird;
c. Berechnung der benötigten Minimumlöslichkeit des Salzes besagter Verbindung, die nötig ist, besagte maximale Gesamtdosis zu formulieren;
d. Erhalt besagter Salzserien;
e. Bestimmung der Gleichgewichtslöslichkeit eines jeden der besagten Salze in besagtem Cyclodextrin und
f. Auswahl besagten nützlichen Salzes, eines Salzes besagter Serien, das in besagtem Cyclodextrin eine genügende Gleichgewichtslöslichkeit aufweist, die es erlaubt, eine Gesamtdosis einzustellen, die gleich oder geringer ist, als die besagte maximale Gesamtdosisdosis.

## Revendications

**1.** Méthode pour établir la présence d'un ou plusieurs sels d'un composé, lesdits sels ayant une solubilité dans une cyclodextrine égale ou supérieure à une solubilité cible désirée, comprenant l'obtention d'une série de sels dudit composé, la détermination de la solubilité à l'équilibre de chaque sel dans ladite série dans une solution aqueuse de ladite cyclodextrine, et la comparaison de chaque solubilité mesurée avec ladite solubilité cible.

**2.** Méthode de détermination d'un sel utile, à partir d'une série de sels d'un composé médicamenteux particulier, à des fins d'utilisation dans la préparation d'une composition comprenant ledit sel et une cyclodextrine, ladite méthode comprenant des étapes consistant :

a. à obtenir ladite série de sels ;
b. à déterminer la solubilité à l'équilibre, dans une solution aqueuse de cyclodextrine, de chacun desdits sels dans ladite série ; et
c. à sélectionner, en tant que ledit sel utile, un sel dans ladite série ayant une solubilité dans ladite solution de cyclodextrine égale ou supérieure à une solubilité cible désirée.

**3.** Méthode de détermination d'un sel utile, à partir d'une série de sels d'un composé médicamenteux particulier, à des fins d'utilisation dans la préparation d'une composition comprenant un complexe d'inclusion dudit sel dans une cyclodextrine, ladite méthode comprenant les étapes consistant :

a. à déterminer une quantité dudit composé médicamenteux requise pour l'efficacité thérapeutique ;
b. à choisir une dose totale maximale d'administration de ladite quantité de composé médicamenteux ;
c. , à calculer la solubilité minimale requise d'un sel dudit composé nécessaire pour formuler ladite dose maximale totale ;
d. à obtenir ladite série de sels ;
e. à déterminer la solubilité à l'équilibre de chacun desdits sels dans ladite cyclodextrine ; et
f. à sélectionner, en tant que ledit sel utile, un sel de ladite série ayant une solubilité à l'équilibre dans ladite cyclodextrine suffisante pour permettre la préparation d'une dose totale égale ou inférieure à ladite dose maximale totale.

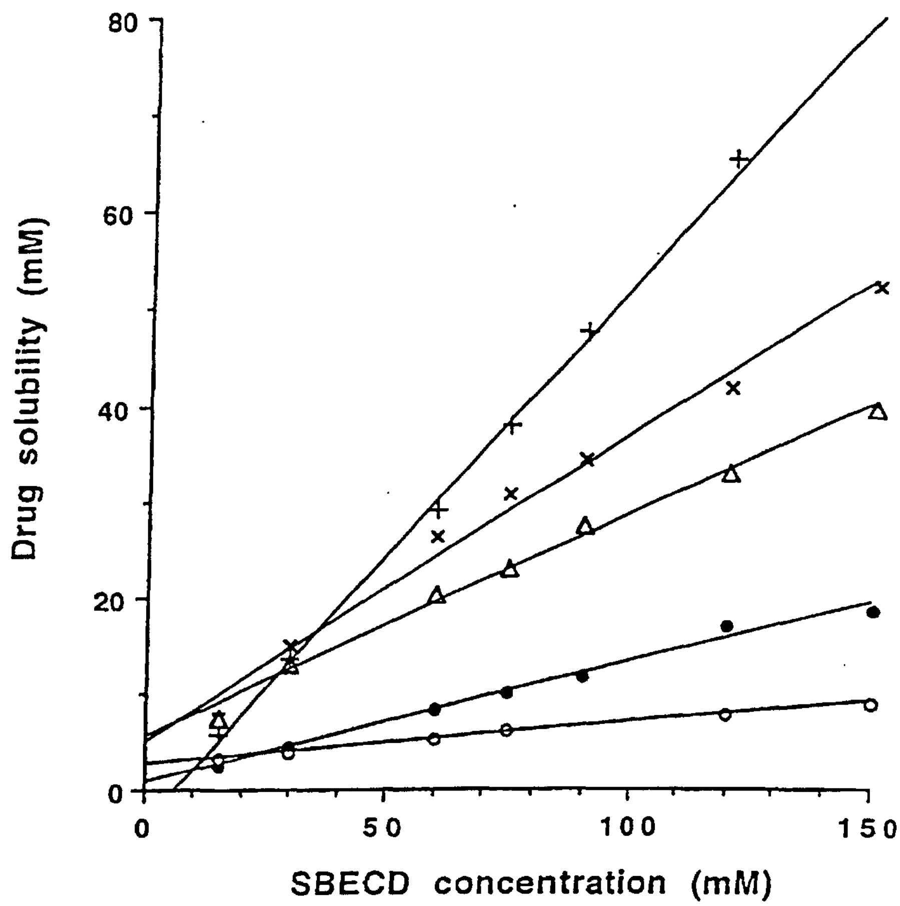
80
60
40
20
0
Drug solubility (mM)
0
50
100
150
SBECD concentration (mM)

Figure 1